# NEUE EUROPÄISCHE PATENTSCHRIFT

(11) **EP 0 499 348 B2**
(45) Veröffentlichungstag und Bekanntmachung des Hinweises auf die Entscheidung über den Einspruch: **27.03.2002**
(45) Hinweis auf die Patenterteilung: 17.04.1996
(21) Anmeldenummer: 92250018.6
(22) Anmeldetag: 22.01.1992
(51) Int. Cl.: A61K 31/57

(54) **Ovulationshemmendes Mittel zur hormonalen Kontrazeption**
Ovulation-inhibiting agent for hormonal contraception
Agent inhibiteur d'ovulation pour contraception hormonale

(30) Priorität: 09.02.1991 DE 4104385
(43) Veröffentlichungstag der Anmeldung: 19.08.1992
(73) Patentinhaber: Ehrlich, Marika, Dr. med., D-55234 Framersheim (DE); Kuhl, Herbert, Prof. Dr., D-63741 Aschaffenburg (DE)
(72) Erfinder: Ehrlich, Marika, Dr. med., D-55234 Framersheim (DE); Kuhl, Herbert, Prof. Dr., D-63741 Aschaffenburg (DE)
(74) Vertreter: Goddar, Heinz J., Dr.

(56) Entgegenhaltungen:
- EP-A- 0 036 229
- EP-A- 0 368 373
- US-A- 3 502 772
- US-A- 3 639 600
- US-A- 3 733 407
- US-A- 4 921 843

## Beschreibung

Die Erfindung betrifft ein ovulationshemmendes Mittel zur hormonalen Kontrazeption, mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachter Hormon-Tageseinheiten bestehen, wobei eine erste der Hormonkomponenten als hormonellen Wirkstoff im wesentlichen ausschließlich ein eine Störung der Follikelreifung bewirkendes Östrogenpräparat, die zweite Hormonkomponente hingegen in Kombination ein Östrogen-und in mindestens zur Ovulationshemmung ausreichender Dosierung ein Gestagenpräparat enthält.

Als hormonale, in Tageseinheiten oral einzunehmende Ovulationshemmer sind einerseits Kombinations- und andererseits Sequenzpräparate bekannt. Bei bekannten Kombinationspräparaten wird beispielsweise, sofern die gewünschte Zyklusdauer 28 Tage beträgt, 21 Tage lang in konstanter oder wechselnder absoluter und/oder relativer Dosierung eine Kombination aus einem Östrogenpräparat und einem Gestagenpräparat verabreicht, wobei das Östrogenpräparat z. B. natürliches Östrogen oder synthetisches Ethinylestradiol sein kann und sich an die Einnahme der vorgenannten 21 Tageseinheiten eine siebentägige Pause anschließt, in der es zu einer die natürliche Monatsblutung simulierenden Entzugsblutung kommt.

Bei den bekannten Sequenzpräparaten wird, wiederum bei einer gewünschten Zyklusdauer von 28 Tagen, 7 Tage lang ein reines Östrogenpräparat und dann 15 Tage lang eine Kombination aus einem Östrogenpräparat und einem Gestagenpräparat verabreicht, wobei sich auch hier wieder eine einnahmefreie Zeit von z. B. 6 Tagen anschließt, in der es zur Entzugsblutung kommt. Es ist zwar bereits bekannt, die den Kombinations- und den Sequenzpräparaten eigene Einnahmepause im Interesse einer größeren Einnahmesicherheit dadurch zu überbrücken, daß innerhalb der betreffenden Tage Placebos verabreicht werden, jedoch ist man bisher stets davon ausgegangen, daß während der etwa einwöchigen Einnahmepause keine Hormone der hier in Rede stehenden Art verabreicht werden dürfen, um eine zuverlässige Entzugsblutung zu gewährleisten. Lediglich bei Substitutionspräparaten hat man während des Gesamtzyklus Hormone verabreicht, beispielsweise in der Abfolge 10 Tage Östrogenpräparat, 11 Tage Kombination aus Östrogen- und Gestagenpräparat, 7 Tage Östrogenpräparat in besonders niedriger Dosierung, jedoch sind diese Substitutionspräparate zur Ovulationshemmung nicht geeignet.

Die bei Substitutionstherapie verwendeten Sequenzpräparate sind zur Kontrazeption insbesondere deshalb ungeeignet, weil das natürliche Östradiol in der gegebenen Dosierung die Ovulation nicht verhindert und die Phase, in der Gestagen verabreicht wird, mit nur elf Tagen zu kurz ist. Die vorstehend beschriebene sequentielle Anordnung gewährleistet bei den Substitutionspräparaten jedoch eine relativ gute Zykluskontrolle.

Allgemein sind die drei wichtigstens Aspekte, die bei der hormonalen Kontrazeption zu beachten sind, die kontrazeptive Sicherheit, eine gute Zykluskontrolle sowie ein Minimum an Nebenwirkungen. Die kontrazeptive Sicherheit beruht in erster Linie auf der Wirkung der Gestagenkomponente; sie wird bei einem bekannten Präparat in einer Dosis eingesetzt, die etwa doppelt so hoch ist wie die zur Ovulationshemmung notwendige Dosis. Dazu kommen die peripheren Wirkungen des Gestagens auf Zervix, Tuben und Endometrium. Demnach ist Gestagen in den modernen oralen Kontrazeptiva in ausreichender Dosierung vorhanden, um eine zuverlässige Kontrazeption zu gewährleisten. Das normalerweise verwendete synthetische Östrogen Ethinylestradiol verstärkt dabei noch den Ovulationshemmeffekt des Gestagens. Die größte kontrazeptive Sicherheit zeigen bislang Ethinylestradiol - Gestagen - Kombinationspräparate, die über drei von vier Wochen, mit einer Einnahmepause von sieben Tagen, eingenommen werden. Die Sequenzpräparate sind hinsichtlich der kontrazeptiven Sicherheit etwas weniger zuverlässig, da während der reinen Östrogenphase, in der beispielsweise sieben Tage lang 50 µg Ethinylestradiol verabreicht werden, die Ovulation nicht bei allen Frauen verhindert wird und außerdem die peripheren kontrazeptiven Effekte des Gestagens fehlen. Die Ovulationshemmdosis (100 % der Frauen) des Ethinylestradiols beträgt nämlich 100 µg täglich.

Während von den bekannten Ovulationshemmern die Kombinationspräparate, wie oben ausgeführt, die größte kontrazeptive Sicherheit bieten, ist die beste Zykluskontrolle (regelmäßige Entzugsblutungen; möglichst wenig Zwischenblutungen) bei der Anwendung von Sequenzpräparaten u. a. der gattungsgemäßen Art gegeben, die - ähnlich wie in einem normalen ovulatorischen Zyklus - durch eine siebentägige (von Gestagen ungehinderte) Einwirkung des Östrogens eine Proliferation des Endometriums bewirken, bevor das z. B. ab achtem Tag hinzukommende Gestagen die weitere Proliferation hemmt und das Endometrium sekretorisch umwandelt. Etwa zwei bis drei Tage nach der letzten Östrogen-Gestagen-Tablette kommt es bei diesen Präparaten, wie bereits dargelegt, zur menstruationsähnlichen Entzugsblutung, während bei Anwendung der Kombinationspräparate die Proliferation des Endometriums von Anfang an reduziert wird, so daß bei letzteren die Zykluskontrolle schlechter ist als bei den Sequenzpräparaten.

Es hat sich gezeigt, daß insbesondere bei Verwendung von Ethinylestradiol als Östrogenkomponente bei Sequenzpräparaten höhere Dosen insbesondere während der ersten Phase notwendig sind, um die kontrazeptive Wirksamkeit zu gewährleisten, die aber wiederum die Gefahr ernsthafter und gefährlicher Komplikationen bzw. Nebenwirkungen (z. B. Thromboembolieen) in sich bergen. Das verwendete Gestagen kann in einigen Fällen diesen Effekt noch verstärken, wobei das Risiko mit steigendem Alter zunimmt und besonders bei Frauen mit einem Alter von mehr als 40 Jahren besonders ausgeprägt ist. Ein Ausweg wäre im Prinzip die Verwendung von Kombinationspräparaten, die anstelle von Ethinylestradiol das natürliche Östrogen Östradiol enthalten, da aus den Erfahrungen mit der Substitutionstherapie bei postmenopausalen Frauen bekannt ist, daß die Behandlung mit Östradiol mit wesentlich geringeren gesundheitlichen Risiken verbunden ist, jedoch ist Östradiol für die Anwendung in Kombinationspräparaten wenig geeignet. Zwar gewährleistet dabei die Gestagenkomponente einen sicheren Kontrazeptionsschutz; da aber Gestagen durch Stimulierung lokaler Enzyme eine verstärkte Inaktivierung des Östradiols im Endometrium verursacht und die Östrogenwirkung auf das Endometrium stark reduziert ist, kommt es relativ häufig zu Zwischenblutungen, mit den bereits beschriebenen nachteiligen Effekten. Im Gegensatz hierzu wird Ethinylestradiol im Endometrium weitaus langsamer metabolisiert und hat dementsprechend eine ausreichende Wirkung auf das Endometrium.

Aus der US-A-4 921 843 ist ein ovulationshemmendes Mittel der gattunsgemäßen Art bekannt, bei dem zwischen der Einnahme der letzten der Hormon-Tageseinheiten, wie Dragees, Tabletten oder dergleichen, der zweiten Hormonkomponente eine Einnahmepause von mindestens einem Tag, die beispielsweise durch ein Placebo überbrückt werden kann, vorgesehen ist, ehe dann eine neue Hormon-Tageseinheit, und zwar die erste der ersten Hormonkomponente des nächstfolgenden Zyklus, eingenommen wird. Dies entspricht der bislang herrschenden Meinung der Fachwelt, wonach nämlich eine Einnahmepause von mindestens einem Tag oder aber eine drastische Reduzierung des wirksamen Östrogenspiegels als unbedingt notwendig angesehen wurde, um eine Enzugsblutung auszulösen. Ein derartiges Absetzen des Östrogens führt jedoch selbst dann, wenn es sich nur um eine eintägige Einnahmepause handelt, zu Veränderungen der Durchbluting, die beispielsweise Anlaß zu Kopfschmerzen (Migräneanfällen) sein können, ferner auch zu kurzfristigen Veränderungen verschiedener Stoffwechselparameter, insbesondere der Hämostase, so daß eine hinsichtlich des Östrogeneinflusses stabile Stoffwechsellage für einen oder mehrere Tage aus dem Gleichgewicht gerät.

Die US-A-3 639 600 beschreibt ein ovulationshemmendes Mittel, bei dem über die gesamte Zyklus dauer ein Östrogen appliziert wird. Die tägliche Östrogen dosis steigt im verlauf des Zyklus stufenweise an und fällt dann am Zyklusende auf eine sehr niedrige Dosis ab, um eine Entzugsblutung auszulosen. Ein gleichmäßigen Östrogenspiegel über die gesamte Dauer des Zyklus wird hierbei ausgeschlossen.

Auch bei einem aus der DE-A-26 45 307 bekannten Mittel zur Behandlung klimakterischer Ausfallerscheinungen wird eine Einnahmepause oder zumindest die Simulation einer Einnahmepause durch zeitweise Verwendung eines besonders schwachen Östrogentyps, der anders als beim gattungsgemäßen Mittel keine ausreichende Störung der Follikelreifung bewirkt, als notwendig angesehen. Insgesamt reichen die dabei verwendeten Hormondosen, insbesondere auch die Dauer der Gestagenphase, bei dem in der vorstehend genannten Druckschrift beschriebenen Mittel zur Kontrazeption nicht aus. In der DE-A-24 31 704 ist ebenfalls ein Mittel zur Linderung klimakterieller Beschwerden beschrieben, bei dem schwankende Östrogenkonzentrationen vorgesehen sind. Die Gestagengaben beginnen dabei erst nach der Zyklusmitte, wodurch keine kontrazeptive Wirkung erzielt werden kann. Außerdem ist zwingend eine hormonfreie Einnahmepause vorgesehen.

In der EP-A-0 368 373 ist ein ovulationshemmendes Mittel beschrieben, bei dem über die gesamte Zyklusdauer ein konstanter Gestagenspiegel vorgesehen ist, dem zyklisch Östrogenphasen überlagert werden. Nachteilig ist dabei, daß eine erhöhte Zwischenblutungsgefahr besteht und die andauernde Gestagenzuführung zwar einen guten kontrazeptiven Effekt hat, jedoch auch eine dauernde gefäßverengende Wirkung mit sich bringt, so daß gerade bei Frauen mit Neigung zu Durchblutungsstörungen, etwa mit steigendem Alter, gesundheitliche Nachteile nicht ausgeschlossen werden können.

Der Erfindung liegt die Aufgabe zugrunde, das ovlulationshemmende Mittel der gattungsgemäßen Art dahingehend weiterzubilden, daß bei hoher kontrazeptiver Sicherheit eine einwandfreie Zykluskontrolle unter zuverlässiger Vermeidung von Zwischenblutungen erreicht und Nebenwirkungen vermieden werden.

Erfindungsgemäß wird diese Aufgabe dadurch gelöst, daß die Gesamtzahl der Hormon-Tageseinheiten gleich der Gesamtzahl der Tage des gewünschten Zyklus, nämlich 28 Tage, ist, daß die erste Holmonkomponente 5 bis 14 und und die zweite Hormonkomponente 23 bis 14 Tageseinheiten umfaßt; daß die Anzahl der Tageseinheiten der ersten Hormonkomponente geringer als die Anzahl der Tageseinheiten der zweiten Hormonkomponente ist; und daß bei Verwendung des Mittels ein gleichmäßiger Östrogenspiegel erreichbar ist.

Dabei kann vorgesenen sein, daß mindestens eines der Östrogenpräparate mindestens einen Bestandteil aus der Ethinylestradiol, Mestranol, andere synthetische Östrogene sowie mindestens einen der vorgenannten Hormonbestandteile nach Einnahme rasch abspaltende Hormonverbindungen umfassenden Gruppe aufweist.

Eine weitere Ausführungsform der Erfindung ist dadurch gekennzeichnet, daß mindestens eines der Östrogenpräparate mindestens einen Bestandteil aus der Östradiol, Östron und/oder andere natürliche Östrogene sowie mindestens einen der vorgenannten Hormonbestandteile nach Einnahme rasch abspaltende Hormonverbindungen umfassenden Gruppe aufweist.

Nach der Erfindung kann auch vorgesehen sein, daß das Gestagenpräparat mindestens einen Bestandteil aus der Progesteron, Chlormadinonacetat, Norethisteronacetat, Cyproteronacetat, Desogestrel, Levonorgestrel, andere natürliche und/oder synthetische Gestagene sowie mindestens einen der vorgenannten Hormonbestandteile nach Einnahme rasch abspaltende Hormonverbindungen umfassenden Gruppe aufweist.

Nach der Erfindung kann auch vorgesehen sein, daß die erste Hormonkomponente höchstens 10 Tageseinheiten umfaßt.

Eine weitere Ausführungsform der Erfindung ist dadurch gekennzeichnet, daß die erste Hormonkomponente 7 und die zweite Hormonkomponente 21 Tageseinheiten umfaßt.

Der Erfindung liegt die überraschende Erkenntnis zugrunde, daß es gelingt, die kontrazeptive Sicherheit sowie die Zykluskontrolle dadurch zu verbessern, daß einerseits durch eine frühzeitige Störung der Follikelreifung innerhalb der bislang üblichen hormonellen Einnahmepause, nämlich durch die dann einzunehmende Östrogenkomponente, eine hormonelle Kontrolle des follikulären Geschehens über den gesamten Zyklus gewährleistet wird. Dadurch, daß in der reinen Östrogenphase ähnlich den natürlicherweise vorkommenden Verhältnissen zunächst ein ausreichender Schleimhautaufbau erfolgt, treten andererseits Zwischenblutungen auch bei geringerer Dosierung der Hormonkomponenten in der eigentlichen Ovulationshemmphase, in der eine Kombination aus Östrogen und Gestagen verabreicht wird, deutlich seltener auf. Die reine Östrogenphase beträgt mindestens fünf Tage und kann im Fall der Verwendung von natürlichem Östrogen auf bis zu zehn Tage, im Fall der Verwendung von synthetischem Östrogen auf bis zu 14 Tage ausgedehnt werden, woran sich dann, je nach gewünschter Zyklusdauer, die im allgemeinen längere Kombinationsphase anschließt.

Es ist von besonderem Vorteil, wie dies Gegenstand einer besonderen Ausführungsform der Erfindung ist, wenn die Anzahl der Tageseinheiten des erfindungsgemäßen Mittels an den natürlichen individuellen Zyklus der betreffenden Frau angepaßt wird, da dann in Bezug auf die Vermeidung unerwünschter Nebenwirkungen ein besonders günstiger Zustand erreicht wird.

Die reine Östrogenphase zu Beginn der erfindungsgemäßen Behandlung ermöglicht eine ausreichende Proliferation des Endometriums. Ein sicherer kontrazeptiver Schutz wird inbesondere dann erzielt, wenn, wie dies erfindungsgemäß vorzugsweise vorgeschlagen wird, das Gestagen in der sich anschließenden Kombinationsphase in den einzelnen Tageseinheiten in der doppelten Ovulationshemmdosis verabreicht wird. Nach Einnahme der letzten gestagenhaltigen Tablette kommt es innerhalb weniger Tage während der nächstfolgenden Östrogenphase zur Entzugsblutung, wobei gleichzeitig durch die Einnahme des Östrogens die erneute Proliferation des Endometriums in Gang gesetzt wird. Im Hinblick auf das gesundheitliche Risiko ist insbesondere diejenige Ausführungsform der Erfindung, bei der lediglich natürliches Östrogen verwendet wird, von großem Vorteil, da hier bei ausreichender kontrazeptiver Sicherheit und Zykluskontrolle besonders geringe Nebenwirkungen zu erwarten sind.

Das ovulationshemmende Mittel nach der Erfindung wird so eingesetzt, daß zur hormonalen Kontrazeption innerhalb des gewünschten Zyklus tageweise aufeinanderfolgend zunächst eine bestimmte Anzahl von Hormon-Tageseinheiten, wie Dragees, Tabletten oder dergleichen, der ersten Hormonkomponente, die als hormonellen Wirkstoff im wesentlichen ausschließlich ein Östrogenpräparat enthält, und daran anschließend der zweiten Hormonkomponente, die in Kombination ein Östrogen- und in mindestens zur Ovulationshemmung ausreichender Dosierung ein Gestagenpräparat enthält, oral verabreicht werden, wobei am Tage nach der Verabreichung der letzten der Tageseinheiten der zweiten Hormonkomponente des betreffenden Zyklus der erste der Tageseinheiten der ersten Hormonkomponente des nächstfolgenden Zyklus verabreicht wird, so daß unter Ausschluß von Einnahmepausen an jedem Tag eine Hormon-Tageseinheit zur Einnahme kommt.

Die bei der Verwendung des ovulationshemmenden Mittels nach der Erfindung auftretenden Blutungen sind mit weniger Blutverlust verbunden und weniger schmerzhaft, bedingt durch die andauernde Östrogengabe, als bei dem ovulationshemmenden Mittel nach dem Stand der Technik, von dem die Erfindung als Gattung ausgeht. Auch die kontrazeptive Sicherheit ist deutlich höher, weil keine einnahmefreien Tage vorliegen, auch nicht ein einziger, an denen bzw an dem durch natürliche Hormonvorgänge ansonsten die kontrazeptive Sicherheit durchbrochen werden könnte. Wegen der andauernden Östrogenzuführung kommt es ferner zu einem durchgehend positiven gefäßerweiternden Effekt, wodurch also Durchblutungsstörungen entgegengewirkt werden kann. Ein weiterer Vorteil des erfindungsgemäßen ovulationshemmenden Mittels besteht darin, daß bei seiner Verwendung das sogenannte Prämenstruelle Syndrom unterdrückt wird, welches bei Absetzen des Östrogen für auch nur einen bzw. wenige Tage wie im natürlichen Zyklusverlauf in nachteiliger Weise auftreten kann.

Der gleichmäßige Östrogenspiegel, der bei Verwendung des ovlulationshemmenden Mittels nach der Erfindung erreicht wird, vermeidet auch den Abfall und Wiederanstieg von Gerinnungsparametern während der hormonfreien Tage bzw. nach Wiederbeginn der Einnahme im nächsten Zyklus, wodurch u.a. das in einem labilen Gleichgewicht befindliche Gerinnungssystem ansonsten gestört würde. Deshalb eignet sich das ovulationshemmende Mittel nach der Erfindung insbesondere auch für Frauen im Alter von mehr als vierzig Jahren, bei denen die Gefahr von Durchblutungsstörungen mit steigendem Alter bekanntlich zunimmt.

Die Erfindung umfaßt auch ein Verfahren zur hormonalen Kontrazeption, welches in der bestimmungsgemäßen Verabreichung des ovulationshemmenden Mittels nach den Patentansprüchen besteht, wobei also ununterbrochen eine kontrazeptiv wirksame Östrogendosis verabreicht wird, welcher zyklisch Gestagengaben überlagert werden.

Nachstehend wird die Erfindung anhand von Ausführungsbeispielen im einzelnen erläutert.

### Beispiel 1:

Zur ovulationshemmenden Behandlung wurde ein Sequenzpräparat verwendet, welches 7 Tageseinheiten mit jeweils 4 mg Östradiol sowie 21 Tageseinheiten mit jeweils 4 mg Östradiol und 1 mg Norethisteronacetat enthielt. Das Mittel wurde über ein Jahr verabreicht und zeigte bei sehr guter kontrazeptiver Sicherheit praktisch keine Nebenwirkungen, wobei Zwischenblutungen deutlich seltener auftraten als bei herkömmlichen niedrig dosierten Präparaten.

### Beispiel 2:

Es wurde ein ovulationshemmendes Mittel verwendet, welches 10 Tageseinheiten mit jeweils 20 µg Ethinylestradiol und 18 Tageseinheiten mit jeweils 20 µg Ethinylestradiol und 150 µg Levonorgestrel enthielt. Die Beobachtungen bei der Verabreichung entsprachen denjenigen von Beispiel 1.

## Patentansprüche

1. Ovulationshemmendes Mittel zur hormonalen Kontrazeption, mit zwei in einer Verpackungseinheit räumlich getrennt konfektionierten, zur zeitlich sequentiellen oralen Verabreichung bestimmten Hormonkomponenten, die jeweils aus einer Anzahl räumlich getrennt und einzeln entnehmbar in der Verpackungseinheit untergebrachter Hormon-Tageseinheiten bestehen, wobei eine erste der Hormonkomponenten als hormonellen Wirkstoff im wesentlichen ausschließlich ein eine Störung der Follikelreifung bewirkendes Östrogenpräparat, die zweite Hormonkomponente hingegen in Kombination ein Östrogen- und in mindestens zur Ovulationshemmung ausreichender Dosierung ein Gestagenpräparat enthält, **dadurch gekennzeichnet, daß** die Gesamtzahl der Hormon-Tageseinheiten gleich der Gesamtzahl der Tage des gewünschten Zyklus, nämlich 28, ist; daß die erste Hormonkomponente 5 bis 14 Tage und die zweite Hormonkomponente 23 bis 14 Tageseinheiten umfaßt; daß die Anzahl der Tageseinheiten der ersten Hormonkomponente geringer als die Anzahl der Tageseinheiten der zweiten Hormonkomponente ist; und eine derartige Dosierung der Hormonkomponenten vorgesehen ist, daß bei Verwendung des Mittels ein gleichmäßiger Östrogenspiegel erreichbar ist.

2. Mittel nach Anspruch 1, **dadurch gekennzeichnet, daß** mindestens eines der Östrogenpräparate mindestens einen Bestandteil aus der Ethinylestradiol, Mestranol, andere synthetische Östrogene sowie mindestens einen der vorgenannten Hormonbestandteile nach Einnahme rasch abspaltende Hormonverbindungen umfassenden Gruppe aufweist.

3. Mittel nach Anspruch 1 oder 2, **dadurch gekennzeichnet, daß** mindestens eines der Östrogenpräparate mindestens einen Bestandteil aus der Östradiol, Östron und/oder andere natürliche Östrogene sowie mindestens einen der vorgenannten Hormonbestandteile nach Einnahme rasch abspaltende Hormonverbindungen umfassenden Gruppe aufweist.

4. Mittel nach einem der vorangehenden Ansprüche, **dadurch gekennzeichnet, daß** das Gestagenpräparat mindestens einen Bestandteil aus der Progesteron, Chlormadinonacetat, Norethisteronacetat, Cyproteronacetat, Desogestrel, Levonorgestrel, andere natürliche und/oder synthetische Gestagene sowie mindestens einen der vorgenannten Hormonbestandteile nach Einnahme rasch abspaltende Hormonverbindungen umfassenden Gruppe aufweist.

5. Mittel nach Anspruch 3 oder 4, **dadurch gekennzeichnet, daß** die erste Hormonkomponente höchstens 10 Tageseinheiten umfaßt.

6. Mittel nach einem der vorangehenden Ansprüche, **dadurch gekennzeichnet, daß** die erste Hormonkomponente 7 und die zweite Hormonkomponente 21 Tageseinheiten umfaßt.

## Claims

1. An ovulation-inhibiting agent for hormonal contraception having two hormonal components which are made up physically separate from one another in a package unit and which are intended to be orally administered sequentially, each component consisting of a number of daily hormone units which are packed in the packaging unit physically apart from one another and are adapted to be removed individually, a first hormonal component containing as hormonal agent substantially exclusively an oestrogen preparation which disturbs follicle maturation whereas the second hormonal component contains in combination an oestrogen preparation and, in a dosage at least sufficient to inhibit ovulation, a gestagen preparation, **characterised in that** the total number of daily hormone units is equal to the total number of the days of the required cycle, namely 28; **in that** the first hormonal component comprises 5 to 14 daily units and the second hormonal component comprises 23 to 14 daily units; **in that** the number of daily units of the first hormonal component is less than the number of daily units of the second hormonal component, and **in that** dosage of the hormonal components is such that when the agent is used a uniform oestrogen level is obtainable.

2. An agent according to claim 1, **characterised in that** at least one of the oestrogen preparations contains at least one component of the group comprising ethinyl estradiol, Mestranol, other synthetic oestrogens and hormonal compounds which separate out at least one of the said hormonal components rapidly after ingestion.

3. An agent according to claim 1 or 2, **characterised in that** at least one of the oestrogen preparations contains at least one component of the group comprising estradiol, oestron and/or other natural oestrogens and hormonal compounds which separate out at least one of the said hormonal ingredients rapidly after ingestion.

4. An agent according to any of the previous claims, **characterised in that** the gestagen preparation contains at least one component of the group comprising progesterone, chloromadinone acetate, norethisterone acetate, cyproterone acetate, Desogestrel, Levonor-gestrel, other natural and/or synthetic gestagens and hormonal compounds which separate out at least one of the said hormonal components rapidly after ingestion.

5. An agent according to claim 3 or 4, **characterised in that** the first hormonal component comprises at most 10 daily units.

6. An agent according to any of the previous claims, **characterised in that** the first hormonal component comprises 7 daily units and the second hormonal component comprises 21 daily units.

## Revendications

1. Agent inhibiteur d'ovulation pour la contraception hormonale, comportant deux composants hormonaux destinés à l'administration orale séquentielle dans le temps et conditionnés séparément dans l'espace dans une unité de conditionnement, qui consistent chacun en un certain nombre de doses unitaires journalières d'hormones logées dans l'unité de conditionnement en étant séparées dans l'espace et de manière à pouvoir être retirées individuellement, l'un des composants hormonaux contenant, en tant que substance active hormonale, pratiquement exclusivement une composition d'oestrogène provoquant une perturbation de la maturation des follicules, le second composant hormonal contenant par contre en association un oestrogène et un progestatif en au moins une dose suffisante pour l'inhibition de l'ovulation, **caractérisé en ce que** le nombre total des doses unitaires journalières d'hormones est égal au nombre total des jours du cycle désiré, à savoir 28 ; **en ce que** le premier composant hormonal comprend de 5 à 14 doses unitaires journalières et le second composant hormonal comprend de 23 à 14 doses unitaires journalières ; **en ce que** le nombre des doses unitaires journalières du premier composant hormonal est inférieur au nombre des doses unitaires journalières du second composant hormonal ; et **en ce qu'**il est prévu un tel dosage des composants hormonaux que, lorsqu'on utilise l'agent, il est possible d'atteindre un taux constant d'oestrogène.

2. Agent selon la revendication 1, **caractérisé en ce qu'**au moins l'une des compositions d'oestrogène comporte au moins un composant choisi dans le groupe comprenant l'éthinyloestradiol, le mestranol, d'autres oestrogènes de synthèse ainsi qu'au moins l'un des composants hormonaux précités, après prise de composés hormonaux se dissociant rapidement.

3. Agent selon la revendication 1 ou 2, **caractérisé en ce qu'**au moins l'une des compositions d'oestrogène comporte au moins un composant choisi dans le groupe comprenant l'oestradiol, l'oestrone et/ou d'autres oestrogènes naturels ainsi qu'au moins l'un des composants hormonaux précités, après prise de composés hormonaux se dissociant rapidement.

4. Agent selon l'une des revendications précédentes, **caractérisé en ce que** la composition de progestatif comporte au moins un composant choisi dans le groupe comprenant la progestérone, l'acétate de chlormadinone, l'acétate de noréthistérone, l'acétate de cyprotérone, le désogestrel, le lévonorgestrel, d'autres progestatifs naturels et/ou synthétiques, ainsi qu'au moins l'un des composants hormonaux précités après prise de composés hormonaux se dissociant rapidement.

5. Agent selon la revendication 3 ou 4, **caractérisé en ce que** le premier composant hormonal comprend au maximum 10 doses unitaires journalières.

6. Agent selon l'une des revendications précédentes, **caractérisé en ce que** le premier composant hormonal comporte 7 doses unitaires journalières et le second composant hormonal comporte 21 doses unitaires journalières.
